# EUROPÄISCHE PATENTANMELDUNG

(11) **EP 1 769 792 A1**
(43) Veröffentlichungstag der Anmeldung: **04.04.2007**
(21) Anmeldenummer: 05021420.4
(22) Anmeldetag: 30.09.2005
(51) Int. Cl.: A61K 31/192, A61K 31/216, A61P 13/10, A61P 13/12

(54) **Verwendung eines beta-3-Adrenozeptor-Agonisten zur Behandlung von Nieren- und Blasenbeschwerden**

(71) Anmelder: Boehringer Ingelheim Pharma GmbH & Co.KG, 55216 Ingelheim am Rhein (DE)
(72) Erfinder: Die Erfindernennung liegt noch nicht vor

(57) **Zusammenfassung**

Diese Erfindung betrifft die Verwendung von beta-3-Adrenozeptor-Agonisten zur Vorbeugung und Behandlung von Nierenschäden und / oder funktionalen Blasenbeschwerden, insbesondere von Blasenbeschwerden neurogenen Ursprungs in Folge von Rückenmarksschädigungen.

## Beschreibung

Diese Erfindung betrifft die Verwendung von beta-3-Adrenozeptor-Agonisten zur Vorbeugung und Behandlung von Nierenschäden und / oder funktionalen Blasenbeschwerden, insbesondere in Folge neurogener Ursachen als Folge von Rückenmarksschädigungen.

### Stand der Technik

Der untere Harntrakt besteht aus der Harnblase, der Harnröhre (Urethra), den entsprechenden Muskeln und den Ligamenten des Halteapparates. Die Aufgabe der Harnblase besteht in der Speicherung des Harns und dessen Entleerung. Für die Erfüllung der Speicherfunktion ist nicht nur die Relaxation des Harnblasenmuskels (Detrusormuskel), sondern auch Verschlussmechanismen durch den Blasenhals, die glatte Muskulatur der Urethra sowie die quergestreifte Muskulatur der Urethra und des Beckenbodens von Bedeutung. Bei der Harnblasenentleerung (Miktion) kontrahiert sich der Detrusormuskel, während sich Urethra und Beckenboden entspannen bzw. der Harnblasenschließmuskel sich öffnet. Diese Vorgänge bedürfen einer komplizierten Steuerung durch das parasympathische, sympathische und somatische Nervensystem. Rückenmarksverletzungen, z.B. Querschnittslähmungen und andere neurogene Ursachen führen dementsprechend zu Fehlfunktionen in diesen Bereichen. Dabei wird häufig der Detrusor instabil. Eine Folge davon kann darin bestehen, dass sich in der Blase hohen Druckwerte aufbauen, die an die Nieren weiter gegeben werden. Bei einem Teil der Patienten entwickelt sich zudem Harninkontinenz. Auch ohne Inkontinenz benötigen diese Patienten eine Behandlung, da eine Schädigung der Nieren durch die hohen Drücke droht. In besonders schwerwiegenden Fällen wird teilweise sogar Inkontinenz in Kauf genommen, indem bei diesen Patienten z.B. der Schließmuskel operativ eingeschnitten werden muss, damit sich die Blase entleeren kann, bevor hohe Drücke aufgebaut werden.

Selektive beta-3-Adrenozeptor-Agonisten werden im Hinblick auf Ihre Eignung für verschiedene Indikationsgebiete diskutiert. Dazu zählen u.a. Obesitas, Diabetes und Harninkontinenz. Seit 1995 ist bekannt, selektive beta-3-Adrenozeptor-Agonisten in der Therapie der Harninkontinenz einzusetzen (EP 0 958 835).

Es wurde nun gefunden, dass beta-3-Adrenozeptor-Agonisten zur Behandlung oder Prophylaxe von Nierenschäden in Folge hoher Drücke in der Blase oder zur Behandlung von Harninkontinenz bei Patienten mit Rückenmarksschädigungen eingesetzt werden können.

### Aufgabe der Erfindung

Der vorliegenden Erfindung liegt die Aufgabe zugrunde, Schädigungen der Niere in Folge hoher Drücke in der Blase zu behandeln oder den Schäden vorzubeugen.

Eine weitere Aufgabe der Erfindung betrifft die Behandlung von Harninkontinenz bei Patienten mit Rückenmarksschädigungen.

Eine weitere Aufgabe der Erfindung betrifft die Behandlung von Harninkontinenz neurogenen Ursprungs in Folge einer Parkinsonerkrankung, einer Alzheimererkrankung, eines Schädelhirntraumas, Multipler Sklerose u.a., bevorzugt in Folge einer Parkinsonerkrankung, einer Alzheimererkrankung oder Multipler Sklerose.

### Beschreibung der Erfindung

Gemäß der vorliegenden Erfindung wird eine neue pharmazeutische Zusammensetzung bereitgestellt, die wenigstens einen beta-3-Adrenozeptor-Agonisten in einer pharmazeutisch wirksamen Menge als aktiven Bestandteil umfasst.

### a) aktive Komponenten

Nachfolgend werden die bevorzugten aktiven Komponenten benannt. In dem Ausmaß, in dem irgendeine pharmazeutisch aktive Verbindung offenbart oder beansprucht wird, ist es ausdrücklich beabsichtigt, dass alle aktiven Metaboliten, die in vivo erzeugt werden, eingeschlossen sind, und es ist ausdrücklich beabsichtigt, dass alle möglichen Stereoisomere oder Tautomere eingeschlossen sind. Ebenfalls eingeschlossen sind pharmazeutisch annehmbare Salze derselben. Beispiele für pharmazeutisch wirksame Salze Für die basischen Verbindungen seien als Säuren zur Salzbildung beispielhaft genannt: Essig-, Benzolsulfon-(Besylat-), Benzoe-, p-Bromphenylsulfon-, Camphersulfon-, Kohlen-, Citronen-, Ethansulfon, Fumar-, Glucon-, Glutamin-, Bromwasserstoff-, Chlorwasser-, Jodwasserstoff-, Isethion-, Milch-, Malein-, Äpfel-, Mandel-, Methansulfon- (Mesylat-), Mucin-, Salpeter-, Oxal-, Pamoa-, Pantothen-, Phosphor-, Bernstein-, Schwefel-, Wein-, p-Toluolsulfonsäure und dergleichen.

In dem Ausmaß, wie es zur Vervollständigung erforderlich ist, werden die Synthese der Verbindungen, für die Stand der Technik angeführt wird, und deren Dosierungen ausdrücklich durch Bezugnahme auf den an der entsprechenden Stelle zitierten Stand der Technik aufgenommen.

Bei dem erfindungsgemäß verwendeten beta-3-Adrenorezeptor-Agonisten handelt es sich bevorzugt um Phenoxyessigsäurederivate. Diese sind bevorzugt aus der folgenden Gruppe nach Formel I ausgewählt: 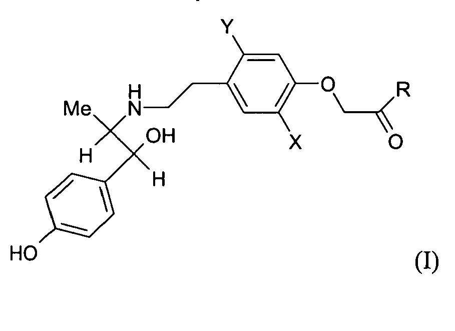 mit
1)X=Br, Y=H, R=OH
   2-[2-Brom-4-[2-[[(1S,2R)-2-hydroxy-2-(4-hydroxyphenyl)-1-methylethyl]amino]ethyl]phenoxy]essigsäure,
2)X=Cl, Y=H, R=OH
   2-[2-Chlor-4-[2-[[(1S,2R)-2-hydroxy-2-(4-hydroxyphenyl)-1-methylethyl]amino]ethyl]-phenoxy]essigsäure,
3) X = Y = C1, R = OH
   2-[2,5-Dichlor-4-[2-[[(1S,2R)-2-hydroxy-2-(4-hydroxyphenyl)-1-methylethyl]amino]ethyl]phenoxy]essigsäure,
4) X = Y = H, R = OH
   2-[4-[2-[[(1S,2R)-2-Hydroxy-2-(4-hydroxyphenyl)-1-methylethyl]amino]ethyl]-2,5-dimethylphenoxy]essigsäure,
5) X = OH; Y = H; R = OH
   2-[2-Hydroxy-4-[2-[[(1S,2R)-2-hydroxy-2-(4-hydroxyphenyl)-1-methylethyl]amino]ethyl]-phenoxy]essigsäure,
6) X = Cl; Y = H, R = OEt
   Ethyl-2-[2-chlor-4-[2-[[(1 S,2R)-2-hydroxy-2-(4-hydroxyphenyl)-1-methylethyl]amino]ethyl]phenoxy]acetat,
7) X = Cl; Y = Cl, R = OEt
   Ethy1-2-[2,5-dichlor-4-[2-[[(1S,2R)-2-hydroxy-2-(4-hydroxyphenyl)-1-methylethyl]amino]ethyl]phenoxy]acetat,
8) X = Me; Y = Me, R = OEt (-)-Ethyl-2-[4-(2-{[(1S,2R)-2-hydroxy-2-(4-hydroxyphenyl)-1-methylethyl]amino}ethyl)-2,5-dimethylphenyloxy]acetat, und das entsprechende Hydrochlorid.
9) X = Me; Y = Me, R = OH
   (-)-2-[4-(2-{[(1S,2R)-2-Hydroxy-2-(4-hydroxyphenyl)-1-methylethyl]amino}ethyl)-2,5-dimethylphenyloxy]essigsäure,

Diese genannten Verbindungen sind in der WO 00/02846 oder der WO 2003024916 offenbart.

Daneben sind folgende Verbindungen von Interesse:
10) 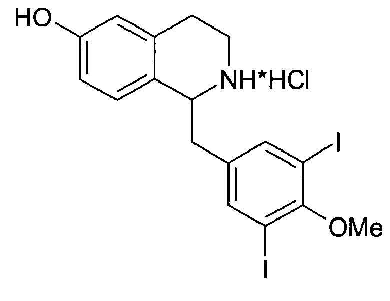 oder die freie Base davon.
   Name: 1-(4-Methoxy, 3,5-diiodophenyl-methyl-1,2,3,4-tetrahydro-isochinolin-6-ol bzw. das Hydrochlorid, J. Med. Chem. 44 (2001) 1456.
11) 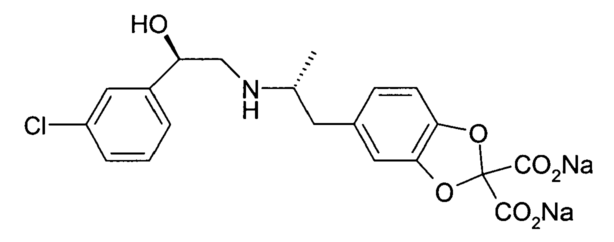 Name: Dmatrium-([R,R]-5-2-[[2-(3-chlorophenyl)-2-hydroxyethyl]-amino]propyl)-1,3-benzodioxol-2,2-dicarboxylat oder das Hydrochlorid, J. Med. Chem. 44 (2001) 1456; Journal of Urology 165 (2001) 240.
12) 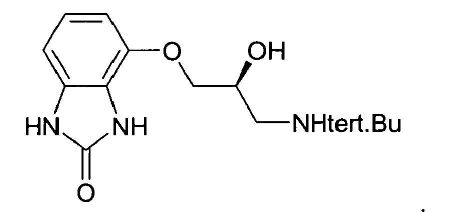 Name: 4-((3-N-tertButylamino)-2-hydroxypropyloxy)-1,3-dihydro-benzoimidazol-2-on oder das Hydrochlorid, Journal of Urology 165 (2001) 240, J. Med. Chem. 44 (2001) 1456.
13) 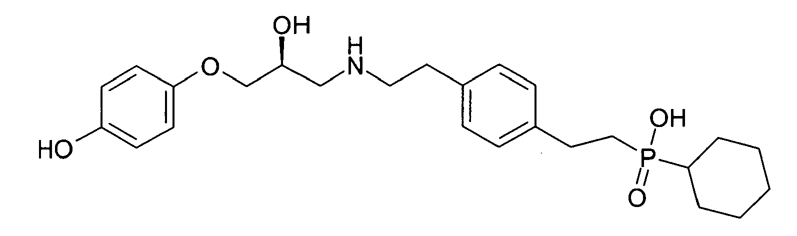 Name: Cyclohexyl-[[4-[2-[[(2S)-2-hydroxy-3-(4-hydroxyl-phenoxy)-propyl]-amino]-ethyl]-phenoxy]-methyl]- phosphinsäure, J. Med. Chem. 44 (2001) 1456.
14) 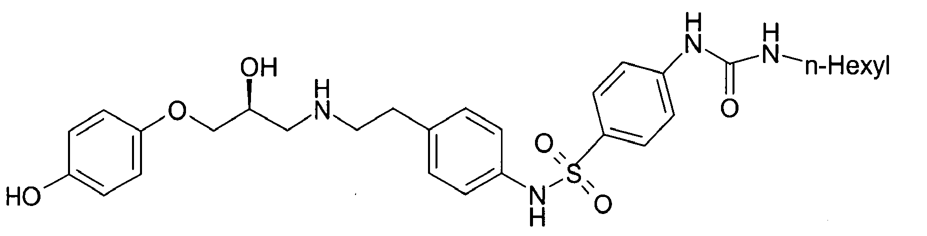 Name: (S)-4-[[(Hexylamino)carbonyl]amino]-N-[4-[2-[[2-hydroxy-3-(4-hydroxy-phenoxy)-propyl]-amino]-ethyl]-phenyl]- benzenesulfonamid, J. Med. Chem. 44 (2001) 1456.
15) 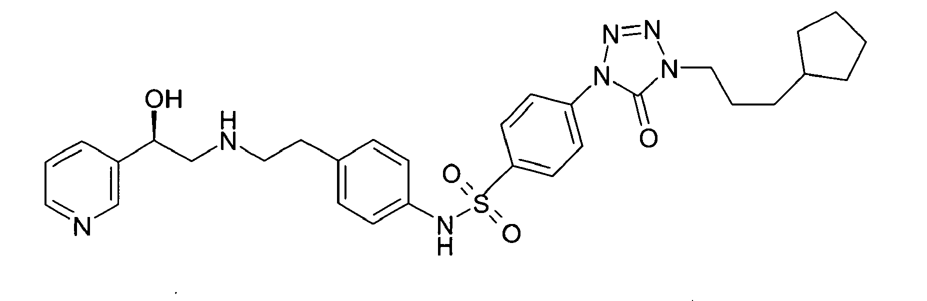 Name: (R)-4-[4-(3-Cyclopentylpropyl)-4,5-dihydro-5-oxo-1H-tetrazol-1-yl]-N-[4-[2-[[2-hydroxy-2-(3-pyridinyl)ethyl]amino]ethyl]-phenyl]- benzenesulfonamid oder das Hydrochlorid, J. Med. Chem. 44 (2001) 1456.
16) 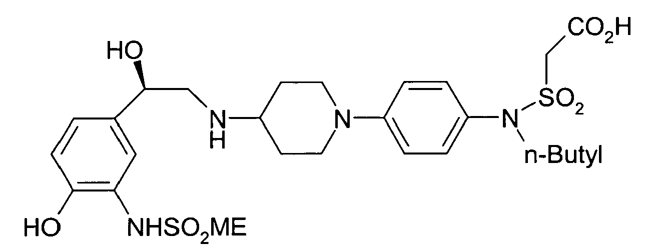 Name: 4-N-(N-(2-(4-Hydroxy-3-methylsulfonamido-phenyl)-2-hydroxy-ethyl-4-amino)-piperidinyl-phenyl-(n-butylamino)-sulfonyl-essigsäure, J. Med. Chem. 44 (2001) 1456, Bioorg. Med. Chem. Lett. 9 (2001) 2045.
17) 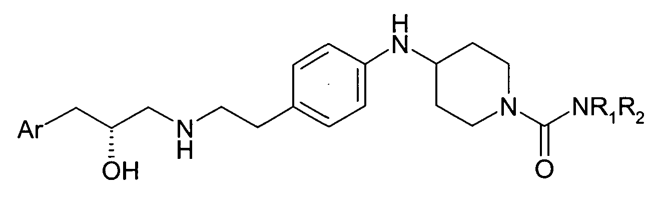 oder das Hydrochlorid mit
   a) Ar = 4-OHPh-O, R1 = Octyl, R2 = H, Name: 4-(4-(2-(3-(4-Hydroxy-phenoxy)-2-hydroxy-propyl)-amino-ethyl)-anilino)-piperidinyl-carbonyl-N-octyl-amid;
   b) Ar = 4-OH, 3-Methylsulphonylamidophenyl-O, R1 = 2,5-difluorbenzyl, R2 = H; Name: 4-(4-(2-(3-(4-Hydroxy-3-methylsulphonylamidophenoxy)-2-hydroxy-propyl)-amino-ethyl)-anilino)-piperidinyl-carbonyl-N-(2,5-difluorobenzyl)-amid;
   c) Ar = 4-OH, 3-Methylsulphonylamidophenyl, R1 = 2,5-difluorbenzyl, R2 = H, Name: 4-(4-(2-(3-(4-Hydroxy-3-methylsulphonylamidophenyl)-2-hydroxy-propyl)-aminoethyl)-anilino)-piperidinyl-carbonyl-N-(2,5-difluorobenzyl)-amid;
   (Bioorg. Med. Chem. Lett. 11 (2000) 3123).
18) 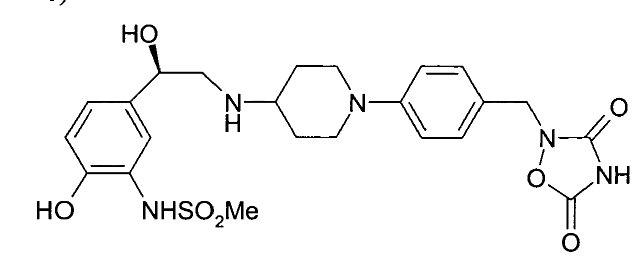 oder das Hydrochlorid
   Name: 2-(4-N-(4-(2-(4-Hydroxy-3-methylsulphonylamidophenyl)-2-hydroxy-ethyl)-amino)-piperidinyl)-benzyl)-[1,2,4]oxadiazolidin-3,5-dion, Bioorg. Med. Chem. Lett. 11 (2001) 981.
19) 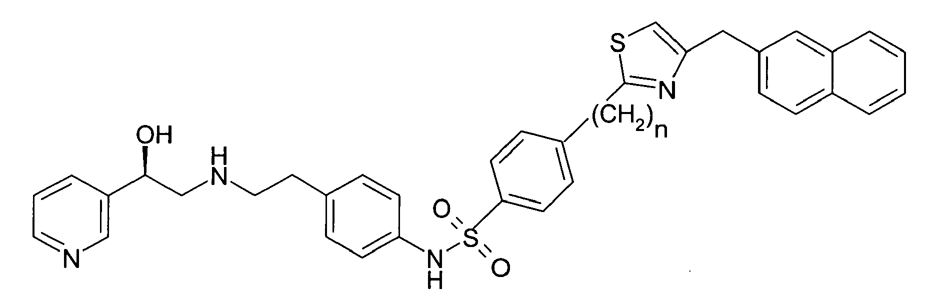 as oder das Hydrochlorid.
   n kann sein 0 oder 1;
   Name: (4-(4-(2-Pyridinyl-2-hydroxyethylamino-ethyl)-anilino)-sulfonyl)-2-benzyl-4-naphth-2-ylmethyl-thiazole (n= 1) and (4-(4-(2-Pyridinyl-2-hydroxyethylamino-ethyl)-anilino)-sulfonyl)-2-phenyl-4-naphth-2-ylmethyl-thiazole (n= 0), Bioor. Med. Chem. Lett. 10 (2000) 1971.
20) 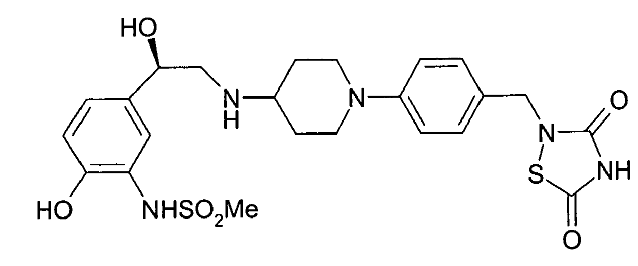 oder das Hydrochlorid.
   Name: 2-(4-N-(4-(2-(4-Hydroxy-3-methylsulphonylamidophenyl)-2-hydroxy-ethyl)-amino)-piperidinyl)-benzyl)-[1,2,4]thiadiazolidin-3,5-dion, Bioorg. Med. Chem. Lett. 11 (2001) 757.
21) 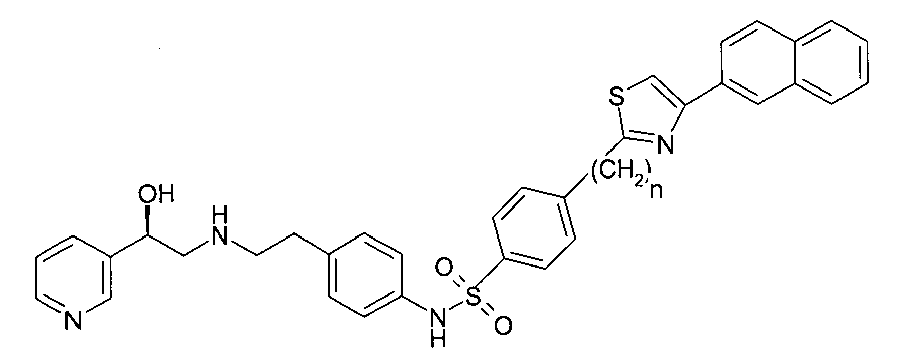 oder das Hydrochlorid.
   n kann sein 0 oder 1.
   Name: (4-(4-(2-Pyridinyl-2-hydroxyethylamino-ethyl)-anilino)-sulfonyl)-2-benzyl-2-naphthyl-thiazol (n= 1) und (4-(4-(2-Pyridinyl-2-hydroxyethylamino-ethyl)-anilino)-sulfonyl)-2-phenyl-2-naphthyt-thiazol (n= 0), Bioor. Med. Chem. Lett. 10 (2000) 1971.
22) 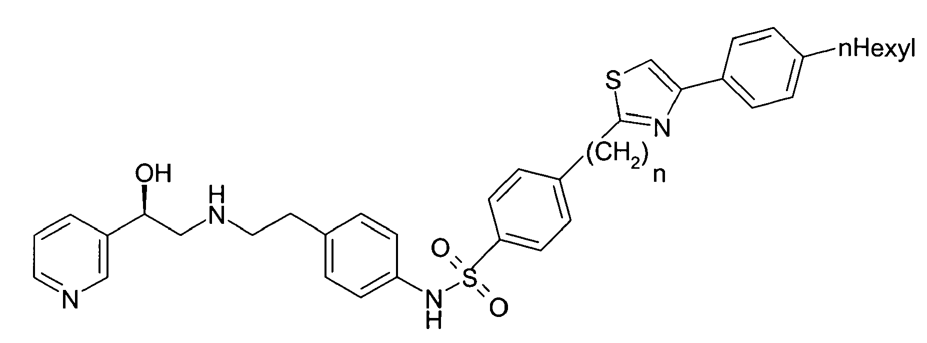 as oder das Hydrochlorid.
   Name: (4-(4-(2-Pyridinyl-2-hydroxyethylamino-ethyl)-anilino)-sulfonyl)-2-benzyl-(4-n-hexyl-phenyl)-thiazol (n= 1) und (4-(4-(2-Pyridinyl-2-hydroxyethylamino-ethyl)-anilino)-sulfonyl)-2-phenyl-(4-n-hexyl-phenyl)-thiazol (n= 0), Bioor. Med. Chem. Lett. 10 (2000) 1971.
23) 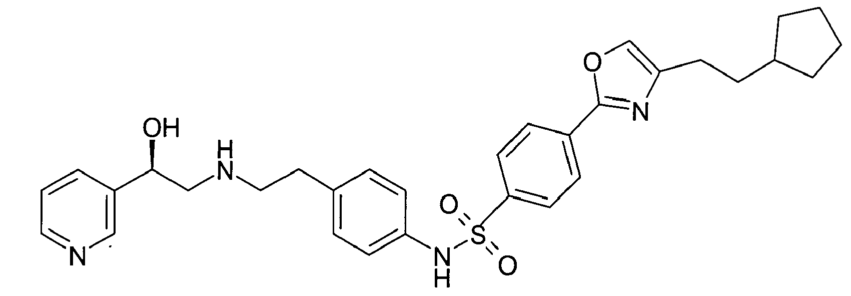 oder das Hydrochlorid.
   Name: 2-(4-(4-(2-Pyridinyl-2-hydroxyethylamino-ethyl)-anilino)-sulfonyl)-phenyl-4-(cyclopentyl-ethyl)-oxazol, Bioorg. Med. Chem. Lett. 10 (2000)1531.
24) 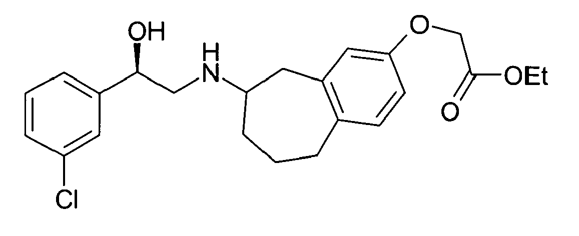 oder das Hydrochlorid.
   Name: Ethyl [R-(R*,S*)]- [[8-[[2-(3-chlorophenyl)-2-hydroxyethyl]amino]-6,7,8,9-tetrahydro-5H-benzocyclohepten-2-yl]oxy]-acetat, hydrochloride,
25) 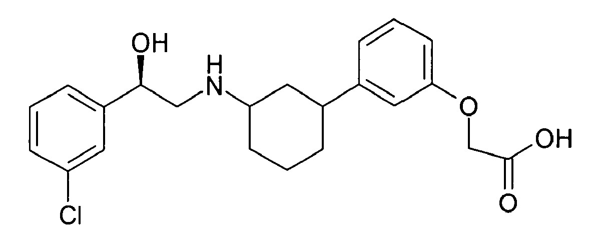 oder das Hydrochlorid.
   Name: [1S-[1α,3β(S*)]]- 3-[3-[[2-(3-Chlorophenyl)-2-hydroxyethyl]amino]cyclohexyl]phenoxy]-essigsäure, Mononatriumsalz,
26) 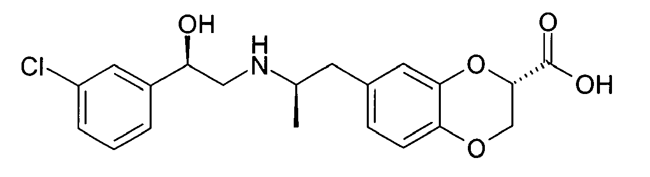 oder das Hydrochlorid.
   Name: 6-[(2R)-2-[[(2R)-2-(3-Chlorophenyl)-2-hydrox-yethyl]-amino]propyl]-2,3-dihydro-(1,4-benzodioxin-2-carboxylsäure).
27)
   2- (3- {[2- (3-Chlorophenyl)-2R-hydroxyl-ethylamino] ethylamino} phenyl) thiophen-3-carboxylsäure oder das Hydrochlorid.
28) 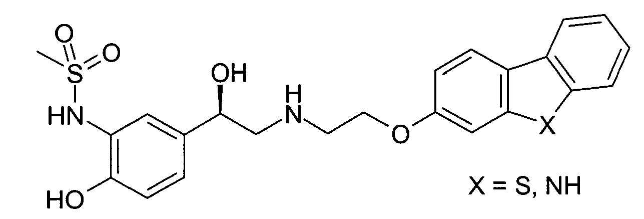 oder das Hydrochlorid.
   Name: 3-(1-(4-Hydroxy-3-methylsolfonylamido-phenyl)-1-hydroxy-ethyl-amino)-ethoxy)-dibenzotiophen und 2-(1-(4-Hydroxy-3-methylsolfonylamido-phenyl)-1-hydroxy-ethylamino)-ethoxy)-9H-carbazol.
29) 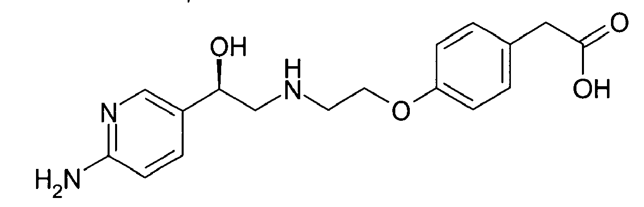 oder das Hydrochlorid.
   Name: [4-[2-[[2-(6-Aminopyridin-3-yl)-2(R)-hydroxyethyl]amino]ethoxy]phenyl]essigsäure.
30) 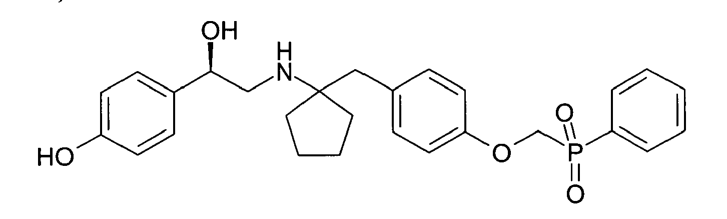 oder das Hydrochlorid.
   Name: [[4-[[1-[[(2S)-2-Hydroxy-3-(4-hydroxyphenoxy)-propyl]-amino]-cyclopentyl]-methyl]phenoxy]-methyl]-phenyl-phosphinsäure.
31) 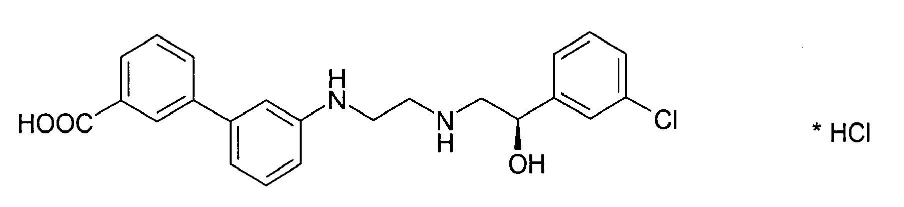 oder die freie Base.
   Name: [1,1'-Biphenyl]-3-carboxylsäure, 3-[[2-[[(2*R*)-2-(3-chlorophenyl)-2-hydroxyethyl]amino]ethyl]amino]- hydrochlorid oderr Solabegron.
32) 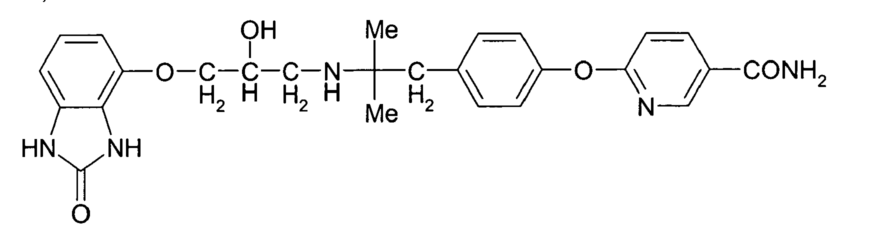 oder das Hydrochlorid.
   Name: 6-[4-[2-[[3-[(2,3-Dihydro-2-oxo-1H-benzimidazol-4-yl)oxy]-2-hydroxypropyl]amino]-2-methyl-propyl]-phenoxy]-3-pyridincarboxamid.
33) 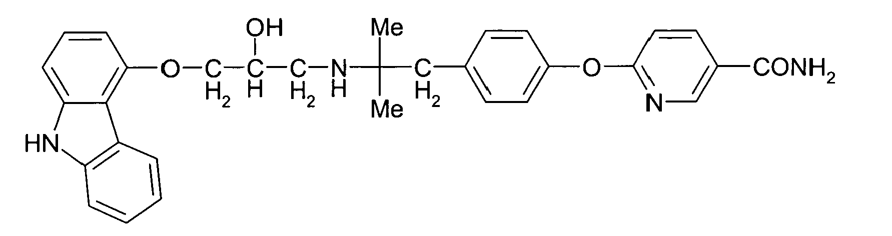 oder das Hydrochlorid.
   Name: (S)-6-[4-[2-[[3-(9H-Carbazol-4-yloxy)-2-hydroxypropyl]amino]-2-methylpropyl]phenoxy]-3-pyridinecarboxamid.

### b) Dosierung

Um die optimale Dosis des Wirkstoffs zu bestimmen, müssen verschiedene Rahmenbedingungen berücksichtigt werden, wie beispielsweise Alter und Körpergewicht des Patienten, Natur und Stadium der Erkrankung, sowie die Potenz der Verbindung. Dies wird als im Vermögen des Fachmanns liegend angesehen, und man kann die bestehende Literatur über die Komponenten zu Rate ziehen, um die optimale Dosierung zu bestimmen. Die angegebenen Dosierungen beziehen sich auf die Dosierung nach Beendigung der Einstellungsphase.

Die im Folgenden angegebenen Dosierungen schließen ausdrücklich alle numerischen Werte, ganze oder gebrochene, innerhalb des angeführten Bereichs ein. Die Angaben beziehen sich auf erwachsene Menschen. Pädiatrische Dosierungen können geringer sein.

Mehr als einmal tägliche oder zweimal tägliche Verabreichungen (z.B. 3, 4, 5 oder 6 Verabreichungen pro Tag) werden ebenfalls ausdrücklich hierin in Betracht gezogen.

In einigen Fällen kann auch eine geringere Menge als die angegebene genügen, während in anderen Fällen eine größere Gesamtmenge notwendig sein kann.

Die tägliche Gesamtdosis kann in Abhängigkeit des Therapieregiments an einem Stück oder innerhalb von mehreren Portionen eingenommen werden. Das Therapieregiment kann auch Abstände zwischen den Einnahmen vorschreiben, die länger als ein Tag sind.

Die durchschnittliche tägliche Dosis des beta-3-Agonisten für einen erwachsenen Mann beträgt etwa 1mg bis 1000 mg, bevorzugt 10 mg bis etwa 750 mg pro Tag, bevorzugt 20 bis 500 mg, stärker bevorzugt 20 bis 200 mg. Diese Menge wird bevorzugt als Einmaldosis oder als Zweimaldosis pro Tag verabreicht.

### c) Applikationsformen

Die Zusammensetzungen der vorliegenden Erfindung können zweckmäßigerweise in einer pharmazeutischen Zusammensetzung verabreicht werden, welche die aktive Komponente in Kombination mit einem geeigneten Träger enthält. Derartige pharmazeutische Zusammensetzungen können durch Verfahren hergestellt werden und Träger enthalten, die in der Technik wohlbekannt sind. Dem Fachmann stehen diesbezüglich allgemein anerkannte Fachwerke zur Verfügung.

Die Zusammensetzungen der vorliegenden Erfindung können parenteral (z.B. durch intravenöse, intraperitoneale, subkutane oder intramuskuläre Injektion), topisch, oral, intranasal, transdermal, rektal, pulmonal inhalativ oder nasal inhalativ verabreicht werden, wobei die orale Verabreichung besonders bevorzugt ist. Unter den oralen Verabreichungsformen können magensaftresistente Formulierungen bevorzugt sein. In diesem Fall sind magensaftresistente Kapseln oder magensaftresistente Tabletten bevorzugt, was in beiden Fällen z.B. mit einem magensaftresistenten Überzug realisiert werden kann. Der Fachmann findet für magensaftresistente Formulierungen im Stand der Technik Anleitungen.

Im folgenden werden verschiedene Formulierungsoptionen gegeben. Der Fachmann kann hieraus eine geeignete Formulierung heraussuchen.

Für die orale therapeutische Verabreichung kann die erfindungsgemäße Zusammensetzung mit einem oder mehreren Trägern vereinigt werden und in Form von einnehmbaren Tabletten, bukkalen Tabletten, Sublingualtabletten, zuckerüberzogenen Tablette, Pulvern, Pudern, Pastillen, Dragees, Granulaten, Kapseln, Elixieren, Suspensionen, Lösungen, Sirupen, Oblaten, Kaugummis, Nahrungsmitteln und dergleichen verwendet werden.

Ein Pulver kann beispielsweise hergestellt werden, in dem die Partikel der aktiven Substanz durch Mahlen auf eine geeignete Größe gebracht werden.
Verdünnte Pulver können dadurch hergestellt werden, dass die pulverförmige Substanz mit einem untoxischen Trägermaterial, wie beispielsweise Laktose fein vermahlen und als Pulver ausgebracht wird. Andere diesbezüglich geeignete Trägermaterialien sind andere Kohlenhydrate, wie Stärke oder Mannitol. Gegebenenfalls können diese Pulver Geschmacksstoffe, Konservierungsstoffe, Dispergierungsagentien, Farbmittel und andere pharmakologische Hilfsstoffe enthalten.

Kapseln können ausgehend von einem Pulver der oben genannten Art oder anderen Pulvern hergestellt werden, die in eine Kapsel, bevorzugt eine Gelatinekapsel, eingebracht werden und die Kapsel danach geschlossen wird.

Es ist auch möglich, dass aus dem Stand der Technik bekannte Schmierstoffe in die Kapsel eingebracht werden oder für den Verschluss der beiden Kapselteile verwendet werden. Die Wirksamkeit einer Kapsel bei oraler Einnahme kann dadurch verstärkt werden, dass disintegrierende oder solubilisierende Stoffe hinzugegeben werden, wie beispielsweise Carboxymethylzellulose, Carboxymethylzellulosecalcium, niedrig substituierte Hydroxyprophylzellulose, Calciumcarbonat, Natriumcarbonat und andere Stoffe. Der Wirkstoff kann in der Kapsel nicht nur als Feststoff, sondern auch suspendiert vorliegen, beispielsweise in Pflanzenöl, Polyethylenglykol, Glycerol mit Hilfe von oberflächenaktiven Substanzen usw.

Tabletten können hergestellt werden, indem die pulverförmige Mischung gepresst wird und anschließend z.B. zu Granulaten weiterverarbeitet wird. Die Tabletten können verschiedene Hilfsstoffe beinhalten, wie z.B. Stärken, Milchzucker, Rohrzucker, Glukose, Natriumchlorid, Harnstoff für Lösungs- u. Injektionstabletten, Amylose, verschieden Zellulosearten wie oben beschrieben und andere.
Als Feuchthaltemitte können beispielsweise Glycerin oder Stärke verwendet werden.

Als Sprengmittel können beispielsweise Stärke, Alginsäure, Calciumalginat, Pektinsäure, pulverisierter Agar-Agar, Formaldehydgelatine, Calciumcarbonat, Natriumbicarbonat, Magnesiumperoxid, Amylose verwendet werden.

Als Gegensprengmittel oder Lösungsverzögerer kommen beispielsweise Rohrzucker, Stearin, festes Paraffin, (bevorzugt mit einem Schmelzbereich von 50-52°C); Kakaofett, hydrierte Fette in Betracht.

Weitere Zerfallsmittel können sein: Maisstärke, Kartoffelstärke, Algininsäure und dergleichen.

Als Resorptionsbeschleuniger eignen sich unter anderem quaternäre Ammoniumverbindungen, Natriumlaurylsulfat, Saponine.

Als Bindemittelverteiler kann z.B. Ether verwendet werden und als Hydrophilisierungsmittel beziehungsweise als Zerfallsbeschleuniger Cetylalkohol, Glycerinmonostearat, Stärke, Maisstärke, Milchzucker, Netzmittel (z.B. Aerosol OT, Pluronics, Tweens), Tragantgummi, Gummi arabicum, Gelatine und andere.

Als Süßungsmittel können Saccharose, Fructose, Lactose oder Aspartam eingesetzt werden oder als Geschmacksmittel Pfefferminz, Wintergrünöl, Kirschgeschmack u.v.m.

Die obige Auflistung ist lediglich beispielhaft, und ein Fachmann könnte andere Hilfsstoffe aus dem Stand der Technik in Betracht ziehen.

Tabletten können beispielsweise durch Direktverpressung hergestellt werden.

Tabletten und ähnliche oral applizierbaren feste Formen können mit Überzügen versehen sein. Zum Beispiel können Tabletten, Pillen oder Kapseln mit Gelatine, Wachs, Schellack oder Zucker und dergleichen beschichtet sein. Wie bereits erwähnt sind für die oralen Darreichungsformen magensaftresistente Formulierungen bevorzugt. Daher sind magensaftresistente Überzüge für Tabletten oder Kapseln bevorzugt. Im Fall eines Sirup oder Elixiers kann Saccharose oder Fructose als Süßungsmittel, Methyl- und Propylparaben als Konservierungsmittel, einen Farbstoff und ein Geschmacksmittel, wie Kirsch- oder Orangengeschmack, enthalten sein.

Auch andere oral applizierbare Formulierungen wie Lösungen, Sirup, Elixier usw. können hergestellt werden. Gegebenenfalls kann die Verbindung mikroverkapselt werden.

Eine parenterale Verabreichung kann dadurch erreicht werden, dass die Verbindung in einer Flüssigkeit gelöst wird und subkutan, intramuskulär oder intravenös injiziert wird. Als Lösungsmittel eignen sich beispielsweise Wasser oder ölige Medien.

Zur Herstellung von Suppositorien kann die Verbindung mit niedrigschmelzenden und wasserlöslichen oder wasserunlöslichen Materialien wie Polyethylenglykol, Kakaobutter, höheren Estern (beispielsweise Moerysthyl, Palmitat) oder Gemischen daraus formuliert werden.

Natürlich sollte jegliches Material, das bei der Herstellung von jeglicher Einheitsdosierungsform verwendet wird, pharmazeutisch annehmbar und in den verwendeten Mengen im wesentlichen nicht-toxisch sein. Zusätzlich können die aktiven Komponenten Präparaten mit verzögerter Freisetzung und Vorrichtungen einverleibt werden, welche, ohne darauf beschränkt zu sein, diejenigen einschließen, die auf osmotischen Drücken beruhen, um ein gewünschtes Freisetzungsprofil zu erzielen. Einmal-täglich-Formulierungen für jede der aktiven Komponenten sind speziell eingeschlossen.

Derartige Zusammensetzungen und Präparate sollten mindestens 0,001 % aktive Verbindung enthalten. Der Prozentsatz der Zusammensetzungen und Präparate kann natürlich variiert werden und kann zweckmäßig zwischen etwa 0,1 bis etwa 100 % des Gewichts einer gegebenen Einheitsdosierungsform ausmachen. Die Menge an aktiver Verbindung in derartigen therapeutisch nützlichen Zusammensetzungen ist derart, dass ein wirksame Dosierungsmenge erhalten wird.

### d) Indikationen

Erfindungsgemäßen Arzneimittelzusammensetzung kann jede der als beta-3-Adrenozeptor-Agonisten gelisteten Verbindungen zur Behandlung oder Prophylaxe von Nierenschäden in Folge des krankhaften oder nicht willentlichen Aufbaus hoher Blasendrücke eingesetzt werden. Daneben kann jede dieser Verbindungen zur Behandlung von Harninkontinenz in Folge von Rückenmarksschädigungen verwendet werden. Die Rückenmarksschädigungen können dabei sowohl eine Folge von Unfällen wie auch von Erkrankungen, z.B. Tumoren sein. Erfindungsgemäß kann auch Harninkontinenz in Folge einer Parkinsonerkrankung, einer Alzheimererkrankung, eines Schädelhirntraumas, Multipler Sclerose behandelt werden. Bevorzugt sind Erkrankungen in Folge einer Parkinsonerkrankung, einer Alzheimererkrankung oder Multipler Sklerose.
Damit umfasst eine weitere Ausführungsform der vorliegenden Erfindung die Verwendung der erfindungsgemäßen Zusammensetzung zur Herstellung eines Medikaments zur Behandlung oder Verhütung einer jeden, der im vorstehenden Paragraphen genannten Indikationen.

Die Behandlung der obigen Krankheiten oder Störungen wird durch Zufuhr einer therapeutisch wirksamen Menge der erfindungsgemäßen Zusammensetzung an einen Säuger bewerkstelligt. In den meisten Fällen ist dies ein Mensch, aber die Behandlung von Nahrungstieren (z.B. Vieh) und Haustieren (z.B. Hunden, Katzen und Pferden) wird ausdrücklich hierin abgedeckt. Für die veterinärmedizinsche Verwendungen können die zu verwendenden Dosierungen andere sein, als die hierin angegebenen Dosierungen.

Es wird erwartet, dass die neue Zusammensetzung mit einem minimalen Grad an schädlichen Nebenwirkungen bei denjenigen für eine rasche Erleichterung sorgen, die an den obigen Krankheiten und Störungen leiden.

### e) Kombinationen

Gemäß der vorliegenden erfindungsgemäße Verwendung kann der beta-3-Adrenozeptoragonist auch mit anderen Wirkstoffen kombiniert werden.

Als bevorzugte Beispiele an Kombinationspartnern für die Behandlung von Harninkontinenz werden genannt:
alpha Blocker wie Tamsulosin, Tamsulosinhydrochlorid, Alfuzosin, Bunazosin, Doxazosin, Indoramin, Naftopidil, Prazosin, Terazosin, Urapidil, Silodosin, Moxisylyt, metazosine, Fiduxosin, Upidosine, SNAP-5089 (5-(N-(3-(4,4-Diphenylpiperidin-1-yl)propyl)carbamoyl) - 2,6-dimethyl-4(R)-(4-nitrophenyl)-1,4-dihydropyridin-3-carboxylsäuremethylester), AIO-8507L,SL-890591((2-(3-(4-(5-chloro-2-methoxyphenyl)piperazine-1-yl)pro pylamino)pyrimidin-4-carboxamid fumarat), RS-100329 (5-methyl-3-(3-(4-(2-(2,2,2-trifluoroethoxy)phenyl)piperazine-1-yl)propyl) pyrimidin-2,4(1H,3H)-dion hydrochlorid);

Antimuscarinica wie (S)-N- {3-[4-(2-(2,3-dihydrobenzofuran-5-yl)-1-methylethyl)-ethylamino]-methyl-piperidin-1-yl]-3-oxopropyl}-methanesulfonamid, [1,1'-biphenyl]-2-ylcarbaminsäure 1-azabicyclo[2.2.2]oct-4-yl-ester monohydrochlorid, 2-methyl-alpha,alpha-diphenyl-1H-imidazol, AH-9700, benzhydryl-carbaminsäure-N-(4-methylamino-benzyl)-piperidin-4-yl ester, Bethancholchlorid, Darifenacin, Darifenacinchlorid, Dicyclominhydrochlorid, Emeproniumchlorid, Fesoterodin, FK-584, Hyoscyaminsulfat, Imipraminhydrochlorid, Oxybutyninchlorid, S-Oxybutyninchlorid, Ipratropium, J-104135, N-[2-(2, 3-dihydrobenzofuran-5-yl)-1-methlethyl]-N-ethyl- (1-methanesulfonylpiperidin-4-ylmethyl)-amin, N-ethyl-N- [2- (4-methoxyphenyl)-1-methylethyl)-[1-(dimethylaminocarbonyl)-piperidin-4-ylmethyl]-amin, Oxybutynin, Propanthelinbromid, Propiverin, Propiverinechlorid, Revatropatchlorid, Solifenacin, Temiverin, Temiverinechlorid, Terodilinechlorid, Tolteridinetartrat, Tolterodin, Trospium, Trospiumchlorid, Vamicamidchlorid.

Für die therapeutische oder prophylaktische Behandlung von Nierenschäden eigenen sich Kombinationspartner wie ACE-Inhibitoren, Angiotensinrezeptorblocker, Renin-Inhibitoren, Calcium-Antagonisten, Diuretika oder beta1- und/oder beta 2 Adrenozeptor-Antagonisten, Endothelin-Rezeptor-Antagonisten. Bevorzugte Kombinationspartner sind ACE-Inhibitoren, Angiotensinrezeptorblocker / Angiotensin II Antagonisten und Diuretika.

Als ACE Inhibitoren seien genannt: Alacepril, Alatriopril, Altiopril Calcium, Ancovenin, Benazepril, Benazepril Hydrochlorid, Benazeprilat, Benzoylcaptopril, Captopril, Captopril-Cystein, Captoprilglutathione, Ceranapril, Ceranopril, Ceronapril, Cilazapril, Cilazaprilat, Delapril, Delaprildiacid, Enalapril, Enalaprilat, Enapril, Epicaptopril, Foroxymithin, Fosfenopril, Fosenopril, Fosenopril Natrium, Fosinopril, Fosinopril Natrium, Fosinoprilat, Fosinoprilsäure, Glycopril, Hemorphin-4, Idrapril, Imidapril, Indolapril, Indolaprilat, Libenzapril, Llisinopril, Lyciumin A, Lyciumin B, Mixanpril, Moexipril, Moexiprilat, Moveltipril, Muracein A, Muracein B, Muracein C, Pentopril, Perindopril, Perindoprilat, Pivalopril, Pivopril, Quinapril, Quinapril Hydrochloride, Guinaprilat, Ramipril, Ramiprilat, Spirapril, Spiraprilhydrochloride, Spiraprilat, Spiropril, Spiropril Hydrochloride, Temocapril, Temocapril Hydrochloride, Teprotide, Trandolapril, Trandolaprilat, Utibapril, Zabicipril, Zabiciprilat, Zofenopril, Zofenoprilat.

Als Angiotensin II Antagonisten seien genannt: Candesartan, Candesartan Cilexetil, Losartan, Valsartan, Irbesartan, Tasosartan, Telmisartan, Eprosartan.

Als Diuretikum sei genannt: Hydrochlorothiazid.

## Patentansprüche

1. Verwendung eines beta-3-Adrenozeptor-Agonisten ggf. in Form eines pharmazeutisch wirksamen Salzes desselben zur Herstellung eines Medikaments zur Prophylaxe und/oder Behandlung von Nierenschädigungen und Nierenerkrankungen als Folge krankhaft hoher Drücke oder durch Fehlfunktion des Detrusors entstandene hohe Drücke in der Blase.

2. Verwendung eines beta-3-Adrenozeptor-Agonisten ggf. in Form eines pharmazeutisch wirksamen Salzes desselben zur Herstellung eines Medikaments zur Behandlung von Blasenerkrankungen in Folge einer Rückenmarksschädigung.

3. Verwendung eines beta-3-Adrenozeptor-Agonisten ggf. in Form eines pharmazeutisch wirksamen Salzes desselben zur Herstellung eines Medikaments zur Behandlung von Blasenerkrankungen in Folge einer Parkinsonerkrankung, einer Alzheimererkrankung, eines Schädelhirntraumas oder Multipler Sklerose.

4. Verwendung eines beta-3-Adrenozeptor-Agonisten ggf. in Form eines pharmazeutisch wirksamen Salzes desselben zur Herstellung eines Medikaments zur Behandlung von Blasenerkrankungen neurogenen Ursprungs.

5. Verwendung nach einem der Ansprüche 1 bis 4, **dadurch gekennzeichnet dass** der beta-3-Adrenozeptor-Agonist eine Verbindungen nach Formel I handelt 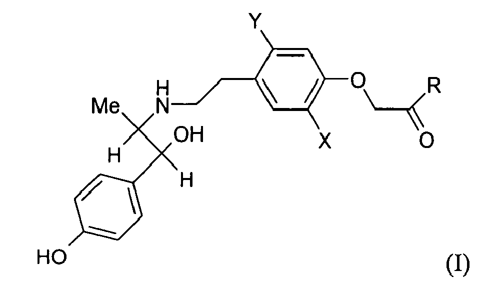 mit X = H, Cl, Br, OH, Methyl, Y = H, Cl, Br, OH, Methyl, R = OH, Methyl, Ethyl oder ein pharmazeutisch akzeptables Salz davon.

6. Verwendung nach Anspruch 5, **dadurch gekennzeichnet, dass**
X = Br, Y = H, R = OH ist.

7. Verwendung nach Anspruch 5, **dadurch gekennzeichnet, dass**
X = Cl, Y = H, R = OH ist.

8. Verwendung nach Anspruch 5, **dadurch gekennzeichnet, dass**
X = Y = Cl, R = OH ist.

9. Verwendung nach Anspruch 5, **dadurch gekennzeichnet, dass**
X = Y = H, R = OH ist.

10. Verwendung nach Anspruch 5, **dadurch gekennzeichnet, dass**
X = OH; Y = H; R = OH ist.

11. Verwendung nach Anspruch 5, **dadurch gekennzeichnet, dass**
X = Cl; Y = H, R = OEt oder das entsprechende Hydrochlorid ist.

12. Verwendung nach Anspruch 5, **dadurch gekennzeichnet, dass**
X = Cl; Y = Cl, R = OEt oder das entsprechende Hydrochlorid ist.

13. Verwendung nach Anspruch 5, **dadurch gekennzeichnet, dass**
X = Me; Y = Me, R = OEt oder das entsprechende Hydrochlorid ist.

14. Verwendung nach Anspruch 5, **dadurch gekennzeichnet, dass**
X = Me; Y = Me, R = OH ist.

15. Verwendung nach einem der Ansprüche 1 bis 14, **dadurch gekennzeichnet dass** der beta-3-Adrenozeptor-Agonist in einer Menge von 10 mg bis 750 mg verwendet wird.

16. Verwendung nach einem der Ansprüche 1 bis 15, **dadurch gekennzeichnet dass** das Medikament zur rektalen, topischen, oralen, sublingualen, intranasalen, transdermalen oder parenteralen Applikation vorgesehen ist.

17. Methode zur Behandlung oder Prophylaxe von Nierenschädigungen und Nierenerkrankungen als Folge krankhaft hoher Drücke oder durch Fehlfunktion des Detrusors entstandene hohe Drücke in der Blase, wobei ein Medikament gemäß einem der vorangegangen Ansprüche verwendet wird.

18. Methode zur Behandlung von Blasenerkrankungen in Folge einer Rückenmarksschädigung, von Blasenerkrankungen in Folge einer einer Parkinsonerkrankung, einer Alzheimererkrankung, eines Schädelhirntraumas oder Multipler Sklerose, wobei ein Medikament gemäß einem der vorangegangen Ansprüche verwendet wird.
